# EUROPEAN PATENT APPLICATION

(11) **EP 2 857 066 A1**
(43) Date of publication of application: **08.04.2015**
(21) Application number: 12877628.3
(22) Date of filing: 29.05.2012
(51) Int. Cl.: A61N 7/00

(54) **METHOD AND APPARATUS FOR TREATING PERIPROSTHETIC CAPSULAR CONTRACTURE**

(71) Applicant: Franco Lissot, Mailin Auxiliadora, 08290 Cerdanyola del Valles (Barcelona) (ES); Arcusa Villacampa, Francisco Javier, 08290 Cerdanyola del Valles (Barcelona) (ES)
(72) Inventor: Franco Lissot, Mailin Auxiliadora, 08290 Cerdanyola del Valles (Barcelona) (ES); Arcusa Villacampa, Francisco Javier, 08290 Cerdanyola del Valles (Barcelona) (ES)
(74) Representative: Morgades y Manonelles, Juan Antonio
(86) International application number: PCT/ES2012/070395
(87) International publication number: WO 2013/178830

(57) **Abstract**

The invention relates to an alternating-current-generating apparatus that uses at least one piezoceramic transducer connected via the corresponding cables in order to transform said current into ultrasound. The purpose of the ultrasonic energy is to eliminate or reduce the periprosthetic capsules of fibrin formed by the human body around the previously implanted prostheses.

The invention also relates to the operating method of the apparatus, used to follow treatment for the destruction or reduction of the capsular contractures.

## Description

### Object of the Invention.

More specifically the invention relates to an alternating-current-generating apparatus that uses at least one piezoceramic transducer connected via the corresponding cables in order to transform said current into ultrasound. The purpose of the ultrasonic energy is to eliminate or reduce the periprosthetic capsules of fibrin formed by the human body around the previously implanted prostheses.

The invention also relates to the operating method of the apparatus, used to follow treatment for the destruction or reduction of the capsular contractures.

### State of the Art.

The inclusion of a foreign body inside the human body results in three responses in reaction thereto: destruction, expulsion or isolation of the body. In the case of female breast prostheses neither of the first two responses is possible, the body reacting through a mechanism whereby a fibrin capsule is created within a few days of placement of the implant or prosthesis.

The histology of the periprosthetic capsule, suffering contracture or otherwise, includes many cellular elements which are dynamic in presentation and number depending on time, the nature of the implant, and the specific response of the recipient body, which is different in each patient.

Many research methods have been developed to better describe the most common elements in the capsular histology, whose common point is the possibility of there being a reaction to the foreign body, with all the corresponding cellular elements, after placement of the implant.

When capsular contracture is resistant to ultrasound treatment, massage or closed capsulotomy, open capsulectomy is the only possible solution.

Invention Patent no. 9701347 concerns Equipment for the treatment of capsular contracture in breast implants and its application method , using a current generator with adjustable frequency and power, using a harness applied directly to the breasts, which can overcome capsular contractures of Grade IV on the Baker scale.

Two separate transducers are placed in the harness, which can vary their position therein, the equipment being capable of treating contractures caused by the application of silicone gel and soybean oil type prostheses and the like.

### Scope of the Invention.

It has a generator designed for use with one or more piezo-ceramic transducers without being restricted to coordinates limited by a harness, and can work on capsular contractures of Grade I to IV on the Baker Scale over the entire surface of the breasts under treatment by a process which, unlike that known by the State of the Art, works sequentially, power being graduated according to the position of the prosthesis: retropectoral or retrogandular.

### Description of the Invention.

The proposed invention, as one of the objects, consists of a current generator capable of working at frequencies of 20 Khz to 10 MHz and with different output powers and/or power densities, for which purpose the generator is connected to one or more piezo-ceramic transducers by one or more wires, by means of a distribution box to distribute the current from the generator.

The ultrasound generator comprises the following regulations:
- Output power of the transducer.
- Pulse length.
- Length of the pause between pulses.
- Working time of each transducer.

The output power of the transducer can be adjusted between 1 and 60 watts, and the working surface of the transducers ranges from 1 to 20 cm2, with two work modes: continuous or in pulses, and with the possibility of working with a power density in each transducer ranging from 1 to 3w/m2, 3w/m2 being the maximum permitted by law.

As another object of the invention, the procedure used by the generator is to use several transducers working sequentially, so that the generator supplies them with a power to fit the Baker Scale from I to IV and depending on the position of the prosthesis and/or implant, while the patient can use a button connected to the generator to interrupt treatment in case of emergency.

Alternatively, the above procedure allows the power supplied to each transducer and the time allocated to each transducer to be different, so the encapsulation treatment allows the operator to make multiple adjustments according to the Baker Scale, and in any position on the chest or on the back and sides.

Other details and characteristics shall be shown throughout the description below referring to drawings attached to this report which are shown for illustrative but not limiting purposes only in a drawing of the invention.

### Description of the drawings.

Below is a list of the different parts of the invention, which are indicated in the drawings attached to this report with their respective numbers; (10) alternating current generator, (11) distribution box, (12) umbilical conductor from the alternating current generator (10), (13 to 16) buttons for adjusting power of the generator (10), adjusting times, sequences and pauses, (17) head of transducer, (18) cable of the transducer, (19) housing of the transducer, (20) vibrating metal part which, in turn, comes into contact with the human body, (21) piezoceramic transducer, input connectors, (23) female terminal, (24) ultrasonic head connectors.
Figure 1 is a overall view of the assembly formed by the alternating current generator (10), the distribution box (11) and the headers (17).
Figure 2 is a top plan view of the distribution box (11) of an ultrasonic generator (not shown in the figures), in one of the faces of which there are the input connectors for connecting the transducers (23).
Figure 3 is a front elevation view of a transducer (17) for receiving the excitation current necessary to generate ultrasounds, through the distribution box (11) and from the current generator (10).

### Description of an embodiment of the invention.

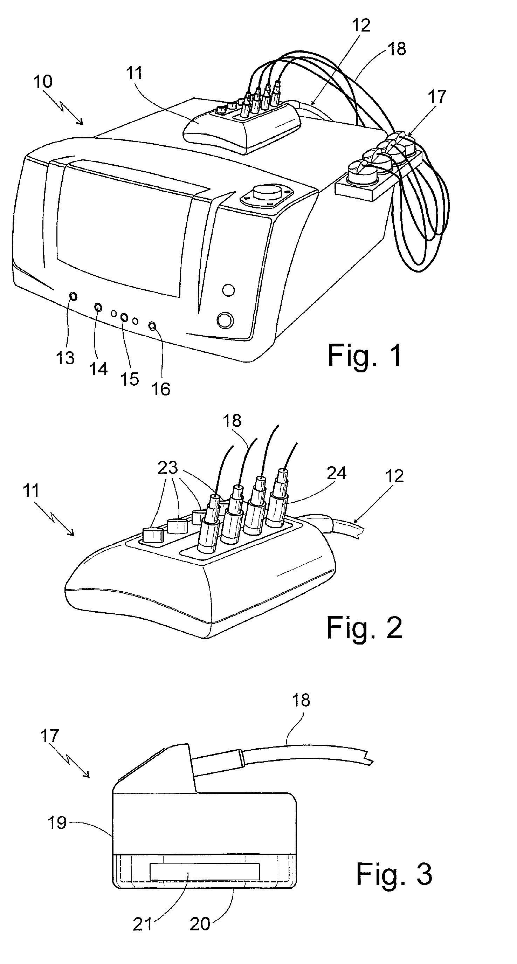

In one of the preferred embodiments of the invention, as shown in Figure 1, the apparatus consists of a conventional current generator (10) which, through the umbilical conductor (12), sends the current generated by the generator (10) to a distribution box (11), all parameters relating to said current being controlled by a control panel located on the front of the generator (10).

The box (11), as shown in Figure 2, is equipped with a set of female terminals (23) to which the corresponding connectors (24) are coupled, so that the current travels from the generator (10) to the transducers (21) through respective individual wires (18), which go from the box (11) to the transducers (21), the buttons (13 to 16) for adjusting power, time, sequence and pauses being located on the generator (10) and the control panel, all this applying to all the transducers (21).

The function of the box (11) is to prevent the wires (18) from the various transducers (21) becoming entangled, so the box (11) in working position rests on the abdomen of the person being treated, facilitating the work of the doctor operating the generator (10), such that, regardless of the number of transducers (21), the generator (10) is connected to the box (11) by a single conduit, which we shall refer to as umbilical (12).

The transducers (21), as shown in Figure 2, have a head (17) with a housing (19) made of ultrasound insulating material and, at the bottom, a vibrating metal part (20) made of titanium, stainless steel or aluminium, while inside the housing (19) there is a piezo-ceramic transducer (21) which transmits the ultrasounds through the vibrating metal part (20) to the patient s skin.

The procedure by which the apparatus works and which makes the treatment of contractures possible starts with the activation of the alternating current generator (10), which sends the current to the distribution box (11) through the umbilical conductor (12), while the generator (10) contains the means of adjustment of all the working parameters of the apparatus, such as the power sent to each transducer (21), the time the selected power is applied in the generator panel (10) by the transducers (21), the times of interruption of power supply to the transducer (21), the work rate of each transducer (21) according to the coordinates of application, since the geometry of each contracture is different, and the geometry of the previously applied prosthesis and/or implant.

Having sufficiently described this invention using the figures attached, it is easy to understand that any modification may be made to the detail which may be deemed to be appropriate, whenever these changes do not alter the essence of the invention summarised in the following claims.

## Claims

1. **METHOD AND APPARATUS FOR TREATING PERIPROSTHETIC CAPSULAR CONTRACTURE** using ultrasound to treat, reduce or prevent the formation of encapsulations around prostheses and/or implants made of silicone gel, soybean oil and the like, **characterised in that** the apparatus consists of an alternating current generator with frequencies ranging from 20 kHz to 10 MHz, the current being transmitted through an umbilical conductor to a distribution box containing inputs for the transducer terminals, and on the ultrasound generator a set of buttons for the output power of all the transducers, application time of an output signal for each transducer, interruption times for all the transducers, and application sequence between one or more transducers.

2. **METHOD AND APPARATUS FOR TREATING PERIPROSTHETIC CAPSULAR CONTRACTURE** according to the claim 1, **characterised in that** the output power of each transducer is between 1 and 60 watts.

3. **METHOD AND APPARATUS FOR TREATING PERIPROSTHETIC CAPSULAR CONTRACTURE** according to the claim 1, **characterised** in that the contact surface of each transducer is between 1 and 20 cm2.

4. **METHOD AND APPARATUS FOR TREATING PERIPROSTHETIC CAPSULAR CONTRACTURE** according to the claim 1, **characterised in that** the work density of each transducer is between 1 and 3 cm2.
